Europäisches Patentamt

European Patent Office

Office européen des brevets

(19)

(11) Veröffentlichungsnummer: **0 498 518 A1**

# (12) EUROPÄISCHE PATENTANMELDUNG

(21) Anmeldenummer: **92250026.9**

(22) Anmeldetag: **10.02.92**

(51) Int. Cl.5: **A61F 2/36**, A61F 2/46

(30) Priorität: **08.02.91 DE 9101657 U**
**22.05.91 DE 9106437 U**

(43) Veröffentlichungstag der Anmeldung:
**12.08.92 Patentblatt 92/33**

(84) Benannte Vertragsstaaten:
**AT BE CH DE DK ES FR GB GR IT LI LU MC NL PT SE**

(71) Anmelder: **Effner GmbH**
**Alt-Lankwitz 102**
**W-1000 Berlin 46(DE)**

(72) Erfinder: **Anapliotis, Emmanuel**
**Kiebitzweg 5**
**W-1000 Berlin 33(DE)**

(74) Vertreter: **Christiansen, Henning, Dipl.-Ing. et al**
**Patentanwalt Pacelliallee 43/45**
**W-1000 Berlin 33(DE)**

(54) **Gelenkprothesensystem.**

(57) Gelenkprothesensystem bestehend aus einem Prothesenschaft, der einen konusförmigen Prothesenhals aufweist, einer Gelenkkugel, die eine kegelförmige Aufnahme aufweist und einem Zwischenelement, das zwischen dem Prothesenschaft und der Gelenkkugel einsetzbar ist und dessen Außenkontur der Innenkontur der kegelförmigen Aufnahme und dessen Innenkontur der Außenkontur des konusförmigen Prothesenhalses entspricht, und im Bereich seiner Stirnfläche mit einer durchgehenden Bohrung versehen ist, wobei die durchgehende Bohrung (8) des Zwischenelements (3, 3', 3'') mindestens über einen Teil ihrer Länge ein Innengewinde (9) aufweist und ein Werkzeug (10, 10') vorgesehen ist mit einem Schaft (11, 11'), der mindestens im Bereich eines seiner Enden mit einem an das Innengewinde (9) der durchgehenden Bohrung (8) angepaßten Außengewinde (12, 12') versehen ist und dessen gegenüberliegendes Ende mit einer Anschlußkupplung zur Drehmomenteinleitung oder einem entsprechenden Handgriff versehen ist.

Fig. 1

Die Erfindung betrifft einen Bausatz für eine Hüftgelenkprothese der im Oberbegriff des Anspruchs 1 angegebenen Art.

Derartige Bausätze für Hüftgelenkprothesen sind aus der DE 40 08 563 A1 und aus der EP 0 382 429 A2 bekannt und bestehen aus einem Prothesenschaft, der einen konusförmigen Prothesenhals aufweist, einer Gelenkkugel, die eine kegelförmige Aufnahme aufweist und einem Zwischenelement, das zwischen dem Prothesenschaft und der Gelenkkugel als Distanzstück einsetzbar ist.

Durch unterschiedlich geformte Zwischenelemente, die an ihren beiden Enden jeweils einen Konusanschluß aufweisen, können der Abstand und die Position der Gelenkkugel in Bezug auf den Prothesenschaft variiert werden.

In den vorgenannten Schriften weisen die Zwischenelemente an ihren Stirnflächen jeweils eine durchgehende Bohrung auf.

Ein Entfernen der Zwischenelemente bei der Demontage, wie sie beim Anpassen zur Auswahl des in der Größe optimalen Zwischenelements oder bei Reoperationen vorkommen kann, ist mittels Werkzeugen relativ schwierig zu bewerkstelligen, da bisher nur die Möglichkeit besteht, entweder mit einem Hakenteil hinter die Hinterkante der Bohrung zu fassen oder aber ein Klemmelement durch Aufspreizen in der Bohrung zu verankern. Dabei besteht jeweils das zusätzliche Problem, daß durch Variation der Länge der Bohrung bei den unterschiedlichen Zwischenelementen, verschiedene Werkzeuge vorrätig gehalten müssen, um für das jeweilige Zwischenelement die optimalen Handhabungsmöglichkeiten zu bieten.

Der Erfindung liegt die Aufgabe zugrunde, bei einem Bausatz für eine Hüftgelenkprothese der eingangs genannten Gattung ein einfaches einheitliche Werkzeug derart auszubilden, daß es ein einfaches Lösen des Zwischenelements unabhängig von dessen Größe und unabhängig davon, ob sein Innenkonus vom konusförmigen Prothesenhals oder sein Außenkonus von der Gelenkkugel gelöst werden soll, ermöglicht.

Diese Aufgabe wird mit den kennzeichnenden Merkmalen des Anspruchs 1 gelöst.

Die Erfindung beruht auf der Erkenntnis, daß sich durch die Ausnutzung einer Gewindesteigung eine einfache Möglichkeit der Krafteinleitung ergibt, da diese eine Übersetzung einer kraftarmen Drehbewegung in eine Hubkraft großer Intensität ermöglicht, welche zum Lösen eines Zwischenelements für eine kurze Wegstrecke erforderlich ist.

Besonders vorteilhaft bei der Erfindung ist die Tatsache, daß die Reaktionskräfte innerhalb der Anordnung: Zwischenelement - Element aus dem das Zwischenelement zu lösen ist, (d.h. Gelenkkugel bzw. konusförmiger Prothesenhals) - Werkzeug verbleiben und nicht manuell durch den Bedienenden übernommen werden müssen. Dieser Umstand ist insbesondere bei festsitzenden Konusanschlüssen von Vorteil.

Das Werkzeug ist insbesondere handlich bedienbar, wenn ein Handgriff vorgesehen ist, der als quergerichter Knebel ausgebildet ist.

Wenn die Länge des Außengewindes des Werkzeugschafts größer als die größte Länge der Bohrung im Zwischenelement zuzüglich des Abstands der äußeren Stirnfläche des Zwischenelements von der gegenüberliegenden Innenfläche der Gelenkkugel bzw. des Abstands von der inneren Stirnfläche des Zwischenelements zur gegenüberliegenden Stirnfläche des am Prothesenschaft angebrachten konusförmigen Prothesenhalses ist, kann dasselbe Werkzeug in beiden möglichen Fällen eingesetzt werden, wenn das Zwischenelement von dem jeweiligen Gegenkonus zu lösen ist.

Bei einer anderen bevorzugten Ausführungsform ist der Werkzeugschaft mit einem Außengewinde versehen, welches am handgriffernen Ende mit einem Richtungssinn ausgebildet ist und am gegenüberliegenden handgriffnahen Ende den entgegengesetzten Richtungssinn aufweist. Die Bohrung im Zwischenelement ist bei dieser Ausführungsform nur über eine Teillänge, ausgehend von der inneren Konusfläche aus, mit einem Innengewinde versehen, daß dem Außengewinde des handgriffernen Endes des Werkzeugschafts angepaßt ist. Über die restliche Länge ist die etwas verbreitete Bohrung gewindelos.

Falls die Gelenkkugel oder das Gegenelement nicht oder nur schwierig manuell zu halten ist - was insbesondere der Fall ist, wenn im Fall von Duokopfprothesen zwei Gelenkkugeln ineinander drehbar gelagert sind und aus der inneren das Zwischenelement entfernt werden soll -, ist es bei der weiteren vorteilhaften Ausführungsformen bevorzugt an der Gelenkkugel an ihrer dem Prothesenschaft zugewandten Seite mindestens eine Ausnehmung oder Anformung zur Einleitung eines Gegenmoments mittels eines weiteren Werkzeugs vorgesehen. Hier kann dann ein entsprechender Schüssel eingreifen, der mit einer geeigneten Nase an einem den Konus umspannenden Bogen mit einem entsprechenden Griff nach Art eines Schlüssels für Fahrrad-Rücktrittbremsnaben versehen ist.

Andererseits kann auch der Schaft des speziell für das erfindungsgemäße System vorgesehenen Drehwerkzeugs mit einem zusätzlichen hebelförmig ausgebildeten und ein ein entsprechendes Innengewinde aufweisendes Gegenhalteelement versehen werden, das drehmomentschlüssig an dem zu entfernenden Zwischenelement anliegt und die Einleitung eines Gegenmoments in das Zwischenelement ermöglicht, um zum Herausheben des Zwischenelements das Werkzeug gegenüber diesem zu Verdrehen. Der Drehmomentschluß zwischen Gegenhaltewerzeug und Zwischenelement erfolgt dabei durch Reibschluß oder durch drehmomentschlüssige Eingriffsmittel.

Hierzu kann das Gegenhalteelement insbesondere an seiner dem Zwischenelement zugewandten Seite eine der benachbarten Stirnfläche angepaßte mit noppenartigen Erhebungen versehene ebene Anlagefläche aufweisen und innerhalb dieser Anlagefläche eine senkrecht zu dieser Fläche gerichtete Bohrung aufweist, mit einem Innengewinde, das dem Außengewinde des handgriffnahen Endes angepaßt ist. Dieses wird dann nach Art einer Kontermutter, nachdem das handgrifferne Ende des Werkzeugschafts vollständig in die Bohrung eingeschraubt worden ist im entgegengesetzten Sinn gegen die benachbarte Stirnfläche des Zwischenelements geschraubt, so daß das verlängerte, mit einem Handgriff versehene hebelförmige Gegenelement ergriffen und zum Halten der Anordnung mit der einen Hand benutzt werden kann, wenn mit der anderen Hand der Knebel gedreht wird.

Wenn die Länge des Außengewindes am handgrifferen Ende des Werkzeugschafts kürzer ist als die kleinste Länge der Bohrung im Zwischenelement zuzüglich des Abstands von der inneren Stirnfläche des Zwischenelements zur gegenüberliegenden Stirnfläche des am Prothesenschaft angebrachten konusförmigen Prothesenhalses ist, kann diese Ausführungsform des Werkzeugs auch in beiden möglichen Fällen, also sowohl zum Lösen eines Zwischenelements von einem Prothesenhals oder von einer Gelenkkugel eingesetzt werden.

Bei einer weiteren bevorzugten einfachen Ausführungsform ist der Handgriff in Form eines Rändelrings ausgebildet von dem ein Schaft, der derart ausgebildet ist, daß er in den kleinsten Innenkonus aller anzuwendenden Zwischenelemente einsteckbar ist und an der Spitze einen Bereich mit einem dem Innengewinde der Bohrung im Zwischenelement angepaßtes Außengewinde aufweist. Durch das Einsetzen und Eindrehen des Werkzeugs in die Bohrung des Zwischenelements kann das Zwischenelement dadurch gelöst werden, daß der Rändelring, nachdem die Spitze des Werkzeugschafts an der Innenwandung der Gelenkkugel oder an der Stirnfläche des konusförmigen Prothesenhalses anliegt, einfach weitergedreht wird.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:

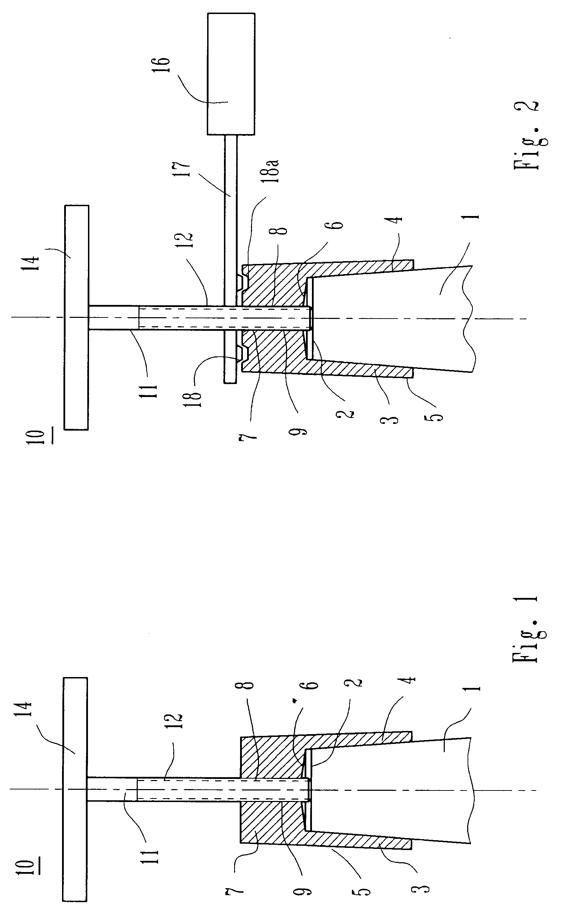

Figur 1 eine Darstellung, welche einen Schnitt durch eine Zusammenstellung von Elementen des erfindungsgemäßen System einschließlich das Werkzeug beim Lösen des Zwischenelements vom Prothesenschaftende,

Figur 2 einen Schnitt gemäß Figur 1 mit einer zweiten Ausführung des erfindungsgemäßen Bausatzes einschließlich Werkzeug beim Lösen des Zwischenelements vom Schaftende,

Figur 3 einen Schnitt durch eine weitere entsprechende Zusammenstellung von Teilen des erfindungsgemäßen Systems, sowie

Figuren 4a und 4b jeweils einen Teilschnitt durch eine weitere Ausführungsform eines Werkzeugs beim Lösen des Zwischenelements aus der Gelenkkugel.

Um zum Aufbringen größerer Kräfte einen Halt für die andere Hand zu haben, ist bei der Ausführungsform gemäß Figur 2 ein hebelförmiges Gegenhalteelement 16 vorgesehen, welches mit seinem Innengewinde 13 auf das Außengewinde 13 des Werkzeugschafts 11 aufschraubbar ist. Das Gegenhalteelement wird auf die Stirnseite des Zwischenelements 3 aufgelegt, so daß seine noppenartigen Vorsprünge 18 - wie dargestellt - in entsprechende Ausnehmungen 18a des Zwischenelements eingreifen. Jetzt kann durch gegenseitiges Verdrehen von Knebel 14 und des hebelförmigem Gegenelements 16 bequem das Zwischenelement 3 vom konusförmigen Prothesenhals 1 gelöst werden, wobei das Ende des Gewindeschafts den Gelenkkonus durch die Gewindewirkung aus dem Zwischenelement herausschiebt.

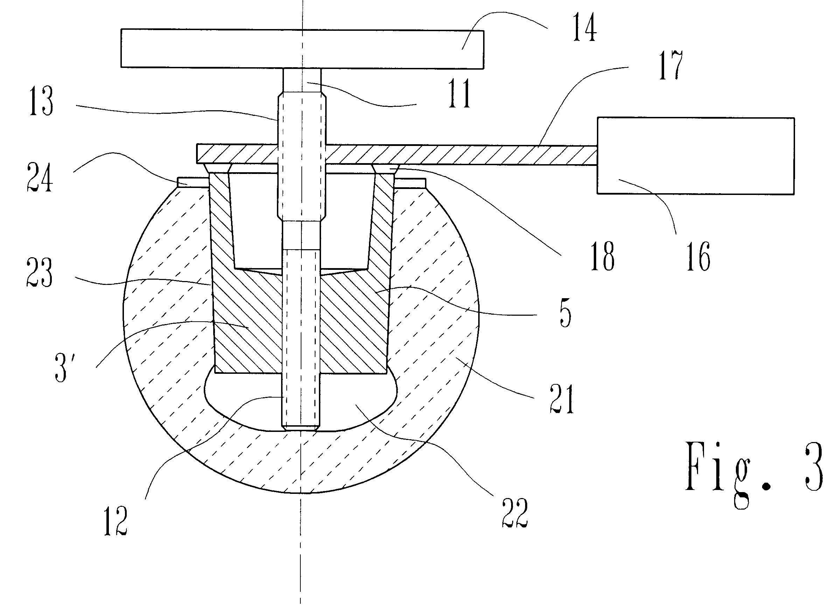

In Figur 3 ist in entsprechender Weise das Lösen des Zwischenelements 3' aus der Gelenkkugel 21 heraus dargestellt. Zum Lösen stützt sich das freie Ende des Werkzeugschafts 11 in diesem Fall auf dem Grunde der Innenausnehmung 22 der Gelenkkugel 21 ab. Das hebelförmige Gegenelement 16 liegt am Stirnflächenbereich des Zwischenelements 3' mit dem größeren Durchmesser an.

Die Bohrung 8 im Zwischenelement 3 ist bei dieser Ausführungsform nur über eine Teillänge, ausgehend von der inneren Konusfläche 6, mit einem Innengewinde 9 versehen, das dem Außengewinde 12 des handgrifferen Endes des Werkzeugschafts 11 angepaßt ist. Über die restliche Länge ist die etwas verbreiterte Bohrung 8 gewindelos. Weiterhin ist ein hebelförmiges, geringfügig elastisches Gegenelement 16 am Werkzeugschaft 11 vorgesehen, das eine mit einem dem Außengewinde des handgriffnahen Endes entsprechenden Innengewinde 20 versehene Bohrung 19 aufweist. Es wird aus der in der Zeichnung wiedergegebenen Position mit einigen Umdrehungen soweit heruntergeschraubt, bis die noppenartigen Erhebungen 18 der unteren Anlagefläche 17 kraftschlüssig an der äußeren Stirnfläche 7 des Zwischenelements 3 anliegen. Jetzt kann ebenfalls durch gegenseitiges Verdrehen von Knebel 14 und hebelförmigem Gegenelement 16 das Zwischenelement 3 vom konusförmigen Prothesenhals 1 gelöst werden, wobei das geringfügig elastische Gegenelement 16 sich verbiegt und das Zwischenelement 3 einen sehr kurzen aber zum Lösen ausreichenden Aufwärtshub erfährt.

Falls ein Gegenhalten mit einem anderen Werkzeug anstelle des hebelförmigen Gegenelements 16 gewünscht wird, sind noch zusätzliche Aussparungen 24 an der die Öffnung für das Zwischenelement 3' aufweisenden Seite benachbart zu dieser Öffnung zur Aufnahme von entsprechenden Drehmomentanschlußmitteln vorgesehen, welche in Form einer Nase an einem - in der Zeichnung nicht dargestellten - den Werkzeugschaft 11 umgreifenden Werkzeug vorgesehen sind.

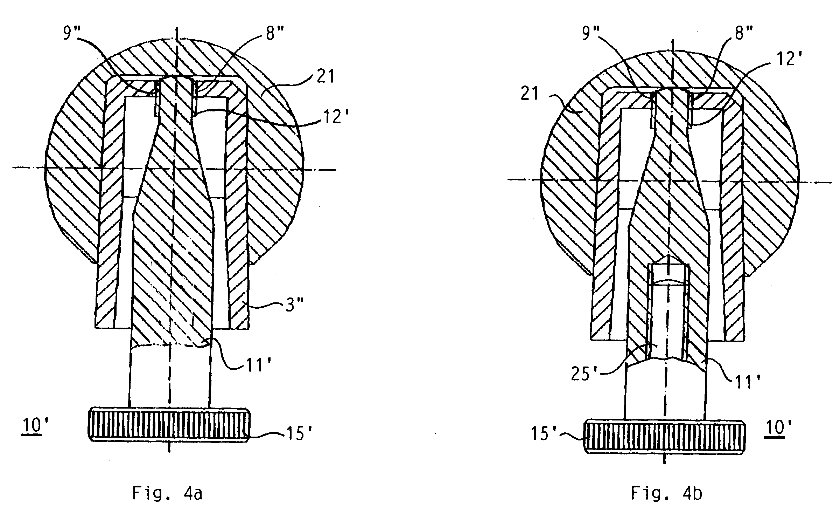

In Figur 4a ist eine weitere Ausführungsform des erfindungsgemäßen Werkzeugs 10' beim Entfernen eines Zwischenelements 3'' aus der Gelenkkugel 21 dargestellt.

Das Werkzeug 10' weist einen als Rändelring 15' ausgebildeten Handgriff mit kreisrundem Querschnitt auf. Der sich vom Rändelring 15' senkrecht erstreckende Werkzeugschaft 11' ist im Spitzenbereich mit einem dem Innengewinde 9'' der Bohrung 8'' im Zwischenelement 3'' angepaßten Außengewinde 12' versehen und kann entweder gemäß der Figur 4a starr oder gemäß der Figur 4b über eine Schraubverbindung 25' mit dem Rändelring 15' verbunden sein. Das Gewinde dieser Schraubverbindung kann gegebenenfalls selbständig zum Lösen eines Zwischenelements verwendet werden. Der Restbereich stellt dabei eine Art aufschraubbare Verängerung 26 dar, so daß jeweils ein Werkzeug optimaler Größe zur Verfügung steht.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch macht.

**Patentansprüche**

1. Gelenkprothesensystem bestehend aus einem Prothesenschaft, der einen konusförmigen Prothesenhals aufweist, einer Gelenkkugel, die eine kegelförmige Aufnahme aufweist und einem Zwischenelement, das zwischen dem Prothesenschaft und der Gelenkkugel einsetzbar ist und dessen Außenkontur der Innenkontur der kegelförmigen Aufnahme und dessen Innenkontur der Außenkontur des konusförmigen Prothesenhalses entspricht, und im Bereich seiner Stirnfläche mit einer durchgehenden Bohrung versehen ist,

   **dadurch gekennzeichnet,**

   daß die durchgehende Bohrung (8) des Zwischenelements (3, 3', 3'') mindestens über einen Teil ihrer Länge ein Innengewinde (9) aufweist und ein Werkzeug (10, 10') vorgesehen ist mit einem Schaft (11, 11'), der mindestens im Bereich eines seiner Enden mit einem an das Innengewinde (9) der durchgehenden Bohrung (8) angepaßten Außengewinde (12, 12') versehen ist und dessen gegenüberliegendes Ende mit einer Anschlußkupplung zur Drehmomenteinleitung oder einem entsprechenden Handgriff versehen ist.

2. Gelenkprothesensystem nach Anspruch 1, **dadurch gekennzeichnet,** daß der Handgriff als quergerichter Knebel (14) oder Rändelring (15') ausgebildet ist.

3. Gelenkprothesensystem nach einem der vorangehenden Ansprüche, **dadurch gekennzeichnet,** daß die Gelenkkugel (21) an ihrer dem Prothesenschaft zugewandten Seite mindestens eine Ausnehmung (22) oder Anformung zur Einleitung eines Gegenmoments aufweist.

4. Gelenkprothesensystem nach einem der vorangehenden Ansprüche, **dadurch gekennzeichnet,** daß die Länge des Außengewindes (12) des Schafts (11) größer ist als die größte Länge der durchgehenden Bohrung (8) im Zwischenelement (3, 3', 3'') zuzüglich des Abstands der äußeren Stirnfläche (7) des Zwischenelements (3, 3', 3'') von der gegenüberliegenden Innenfläche der Gelenkkugel (21) bzw. des Abstands von der inneren Stirnfläche (6) des Zwischenelements (3, 3', 3'') zur gegenüberliegenden Stirnfläche (2) des am Prothesenschaft angebrachten konusförmigen Prothesenhalses (1) ist.

5. Gelenkprothesensystem nach einem der vorangehenden Ansprüche, **dadurch gekennzeichnet,** daß an dem Werkzeug (10) ein hebelförmiges Gegenhalteelement (16) vorgesehen ist, das an seiner dem Zwischenelement (3, 3', 3'') zugewandten Seite eine der benachbarten Stirnfläche (7) angepaßte ebene Anlagefläche (17) aufweist und innerhalb dieser Anlagefläche (17) eine senkrecht zu dieser Fläche gerichtete Bohrung (19) aufweist, mit einem Innengewinde (20), das dem Außengewinde (13) des mit einer Anschlußkupplung zur Drehmomenteinleitung oder einem Handgriff versehenen Ende des Schafts (11) angepaßt ist.

6. Gelenkprothesensystem nach Anspruch 5, **dadurch gekennzeichnet,** daß Drehmomentübertragungsmittel (18) vorgesehen sind, welche bei axialer Verschiebung des Gegenhalteelements (16) in Richtung auf das Zwischenelement (3) mit dessen entsprechenden Gegenelementen (18a) in Eingriff gelangen.

7. Gelenkprothesensystem nach Anspruch 6, **dadurch gekennzeichnet,** daß die Drehmomentübertragungsmittel durch, insbesondere noppenartigen, Erhebungen (18) gebildet werden, welche mit entsprechenden, Gegenelemente bildenden Ausnehmungen (18a) des Zwischenelements in Wechselwirkung treten.

8. Gelenkprothesensystem nach einem der Ansprüche 5 bis 7, **dadurch gekennzeichnet,** daß das Außengewinde (13) des Schafts (11, 11'), ausgehend von seinem einen Ende, mindestens über einen Teil seiner Länge einen entgegengesetzten Richtungssinn aufweist als der vom gegenüberliegenden Ende ausgehende Teil, wobei der am Handgriff gelegene Teil an das Innengewinde der durchBohrung des, insbesondere geringfügig nachgiebig gelagerten, hebelförmigen Gegenhalteelements (16) angepaßt ist, und insbesondere die Länge des an das Innengewinde (9) der durchgehenden Bohrung (8) angepaßten Außengewindes (12) des Schafts (11) kleiner ist als die kleinste Länge der durchgehenden Bohrung (8) im Zwischenelement (3, 3', 3'') zuzüglich des Abstands von der inneren Stirnfläche (6) des Zwischenelements (3, 3', 3'') zur gegenüberliegenden Stirnfläche (2) des am Prothesenschaft angebrachten konusförmigen Prothesenhalses (1).

9. Gelenkprothesensystem nach einem der vorangehenden Ansprüche, **dadurch gekennzeichnet,** daß das der mit einem Gewinde versehene Schaft (25') des Werkzeugs durch einen aufschraubaren Aufsatz (26) verlängerbar ist.

Fig. 2

Fig. 1

Fig. 3

Fig. 4b

Fig. 4a

8

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X,D | EP-A-0 382 429 (SMITH & NEPHEW RICHARDS INC.) | 1,2 | A61F2/36 |
| Y | * Spalte 10, Zeile 52 - Spalte 11, Zeile 7; | 3,4 | A61F2/46 |
| | Abbildungen 1,5,12,18 * | | |
| | --- | | |
| D,X | FR-A-2 644 342 (BRISTOL-MYERS) | 1 | |
| Y | * Zusammenfassung * | 3 | |
| | * Seite 10, Zeile 25 - Seite 11, Zeile 4; | | |
| | Abbildungen * | | |
| | --- | | |
| Y | WO-A-8 907 917 (BIOCONCEPTS, INC.) | 4 | |
| | * das ganze Dokument * | | |
| | --- | | |
| A | US-A-4 100 626 (WHITE) | 5-8 | |
| | * Spalte 3, Zeile 57 - Spalte 4, Zeile 11; | | |
| | Abbildungen 1,2 * | | |
| | --- | | |
| A | WO-A-8 906 116 (BOEHRINGER MANNHEIM CO.) | | |
| | --- | | |
| A | EP-A-0 385 572 (OSTEONICS CO.) | | |
| | ----- | | |

**RECHERCHIERTE SACHGEBIETE (Int. Cl.5 )**

A61F

Der vorliegende Recherchenbericht wurde für alle Patentansprüche erstellt

| Recherchenort | Abschlußdatum der Recherche | Prüfer |
|---|---|---|
| DEN HAAG | 30 MAERZ 1992 | SANCHEZ Y SANCHEZ J. |